(19) Europäisches Patentamt / European Patent Office / Office européen des brevets

(11) **EP 2 130 487 A1**

(12) **EUROPEAN PATENT APPLICATION**

(43) Date of publication:
**09.12.2009 Bulletin 2009/50**

(51) Int Cl.:
*A61B 5/00* (2006.01)   *G06F 19/00* (2006.01)
*G01N 33/487* (2006.01)

(21) Application number: **08104228.5**

(22) Date of filing: **03.06.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Microlife Intellectual Property GmbH 9443 Widnau (CH)**

(72) Inventors:
• **Lin, Kin-Yuan**
  **Taipei (TW)**
• **Huang, Li-Chih**
  **Shulin City (TW)**

(74) Representative: **Hepp, Dieter et al**
  **Hepp, Wenger & Ryffel AG,**
  **Friedtalweg 5**
  **9500 Wil (CH)**

(54) **Glucose meter, a system with a glucose meter, method for operating a glucose meter and a computer program product**

(57)    A glucose meter (10) for measuring the glucose level in a blood sample of a user comprises a memory for storing at least two measurement results of a glucose level measurement. The glucose meter (10) further comprises a display (12) for providing the user with user instructions. The glucose meter (10) can be operated in an OGTT operation mode. In the operation mode, the user is first instructed to make a first measurement. Subsequently, the measurement results are stored in the memory. Then the user is instructed to take a pre-defined drink of glucose. After a pre-determined period of time, on said display (12), instructions to carry out a second measurement are automatically displayed. The result of the second measurement is stored in the memory of said glucose meter (10).

FIG.1

## Description

The invention relates to a glucose meter for measuring the glucose level in a blood sample of a user and to a method for operating such a glucose meter, to a system with such a glucose meter and a computer program product.

The quantitative determination of analytes, in particular the glucose level in the blood is of great importance in the diagnosis and in the continuous control of diseases such as diabetes. In particular, individuals suffering from diabetes should regularly check the glucose level in their blood in order to adopt the glucose intake.

It is well known to check the blood glucose level with biosensors on the basis of a sample of blood. The measurement principle is well known in the art.

Such self-monitoring home blood glucose meters have some drawbacks. In particular, they only provide the blood glucose measurements either at the time of fasting or after a meal. However, the measurement results largely depend on the time when the measurement is made as compared to the time of glucose intake. Users therefore can get confused and may have difficulties to properly interpret the measurement results.

WO2007/019384 discloses such a biosensor which has a post meal test time alarm. The meter includes a user input mechanism which is adapted to allow the user to activate a so-called post meal test time alarm indicating the user to make a measurement at the specific time period after having taken a meal.

EP-1 733 677 is directed to a biosensor and a meter for measuring the glucose level in the blood. The device has an alarm feature reminding the user to make a measurement at predetermined times.

US2003/0211617 also discloses a blood glucose meter that will remind the user to test the blood glucose after a programmable waiting time when a hypoglycemic event is detected.

US-7 241 256 discloses a glucose meter offering a possibility to enter lifestyle data which will be stored in a memory. In particular a specific time and food categories related to a meal can be selected.

US-7 228 163 discloses a glucose meter having the possibility to enter a record of caloric intake.

Insulin insufficiency or insulin resistance may lead to a too high blood glucose level. A so-called oral glucose tolerance test (OGTT) is normally used to determine if a patient is diabetic or pre-diabetic. When carrying out the OGTT, the user is instructed to take a 75g glucose drink and to measure the blood glucose levels at 2 hours after the intake of the drink. The patient is found to be diabetic if the blood glucose level measured 2 hours after the intake of the drink is above 200mg/dl. The patient is considered to be pre-diabetic and having an impaired glucose tolerance if the blood glucose level is between 140 and 200mg/dl measured 2 hours after the drink intake. The OGTT result is a good indication for early detection and early intervention in case of pre-diabetic or diabetic patients.

The OGTT method is generally accepted to be a good method and tool for detecting diabetes and prediabetes. It provides a standardised amount of glucose to be consumed by a user. Healthcare professionals are able to know the users physical insulin response about 2 hours after intake of such amount of glucose. Therewith, it can be determined if a user is diabetic or prediabetic. OGTT are, however not done on a self monitoring basis.

It is further known to use the so-called $Hb_{A1c}$ (Glucosecylate-haemoglobin) test for long term mapping of the average serum glucose. It is generally suggested that diabetic patients should have a $Hb_{A1c}$ test done about 3 to 4 times a year. While it is generally accepted that $Hb_{A1c}$ tests are important tools, such tests have some drawbacks. A number of conditions can lead to falsely elevated or falsely low $Hb_{A1c}$ levels. Alternative methods of monitoring diabetes such as home or office blood glucose measurements are therefore used on a wide scale. In particular, self-monitoring of glucose is widely used in care plans of many people who have type 2 diabetes. Such monitoring is often used to complement $Hb_{A1c}$ measurements and is particularly advantageous because it provides real time feedback of blood glucose levels.

It is therefore an object of the invention to overcome the drawbacks of the prior art, in particular to provide a glucose meter which allows the user to get a better understanding of the meaning of the measurement result and which provides a reliable measurement of the glucose level of the user. A further object of the invention is to provide a glucose meter which can help the users or the healthcare professionals to understand the relation between specific measurements and the times of meal intake. The glucose meter furthermore shall allow the user and/or health care professional to better manage the blood glucose level.

According to the present invention, these and other objects are solved with a glucose meter and a method for operating a glucose meter as well as with a system and a computer program product according to the independent patent claims.

The glucose meter for measuring the glucose level in a blood sample of a user in accordance with the present invention is intended for measurements of the glucose level in a sample of blood applied to a biosensor which can be inserted into the glucose meter. Such meters are known in the art and are not disclosed in more detail. By way of example, such a biosensor is disclosed in WO91/09139.

The glucose meter of the present invention comprises a memory for storing at least two measurement results of a glucose level measurement made at a specific time and the associated time when the measurement has been made. The glucose meter further comprises an instruction interface for providing the user with specific instructions. This instruction interface typically can be a display. It could, however, also be formed as an audible instruction interface.

The glucose meter is adapted to be operated in a so-called OGTT operation mode. When the glucose meter is operated in this OGTT operation mode, the following steps are carried out:

- in a first step, user instructions instructing the user to carry out a first measurement of the glucose level are outputted. This step, however, is optional. It is also possible, that instead of instructing the user to make a first measurement, a measurement routine is automatically started when the operation in the OGTT mode is started.

- The measurement values of such a first measurement of the glucose level together with the associated time and/or date when the measurement has been made are then stored in the memory of the glucose meter, though this may not be necessary in all cases.

- After a pre-determined time, user instructions to carry out second measurement are automatically outputted on said user interface.

- The measurement values of the glucose level obtained in the second measurement are then stored in the memory of the glucose meter. Preferably also the time and/or date associated with the second measurement are stored, though this may not be necessary in all cases.

- Such a glucose meter allows to carry out a OGTT on a self-care basis. If the pre-determined time is e.g. two hours, the user can take a pre-determined glucose drink (e.g. 75g glucose) and make a measurement. After the appropriate time, the user is reminded to make a second measurement. Based on these two measurement results, a reliable OGTT determination can be made.

According to a preferred embodiment of the invention, the glucose meter is further adapted to output user instructions to take a specified amount of glucose, in particular glucose drink. Typically, this instruction can be displayed immediately after making the first measurement.

According to a further preferred embodiment, the glucose meter can be operable in a so-called normal measurement mode. In this measurement mode, a single measurement or a series of single measurements of a glucose level is made. In this context, the glucose meter may be advantageously provided with switching means for switching between the OGTT mode and the normal measurement mode. In particular, the switching means may be formed by an operating button for starting the OGTT measurement. If this OGTT button is not pressed or activated, by default the device operates in a normal measurement mode. Switching by means of operation of a touch screen is also possible.

Preferably, the glucose meter according to the present invention can be further provided with a meal time input interface which is operatively connected with processing means of the glucose meter. The processing means are operating in such a way that upon activation of the meal time input interface, the current time and/or date is stored in the memory. The input interface typically can be a single button. Alternatives such as an interface on a touch screen are conceivable. When the button is pressed, the actual time and/or date is stored. This interface can be used as a means for storing the time and/or date when a meal is taken.

It is furthermore advantageous to display and/or store information in context with the time of measurement. In particular, it can be automatically determined on the basis of activation of the meal time input whether a specific measurement has been made after or before a meal intake. This information can be stored and displayed together with the measurement result. Typically, measurements made within two hours after activation of the meal time input are considered to have been made after meal. While such a feature is preferred in combination with a device operable in the OGTT mode as mentioned above, it is apparent that a glucose meter with a meal time input interface having such additional information possibilities might be advantageous as such. Furthermore, the glucose meter may be provided with means for forming averages of several measurement results. In particular, averages can be formed for after meal and before meal measurements, respectively.

It is possible to repeat the measuring and storing steps at least once when the glucose meter is operating in the OGTT mode. This leads to even more reliable results. In particular, the measurements can be made at shorter times, e.g. during 30 minute intervals, so that during the two hours after intake of the glucose drink, four measurements can be made.

The glucose meter further can be provided with a memory which is adapted to store the values of a plurality of glucose level measurement made on a plurality of different days. Preferably, the glucose monitor in this case can comprise processing means which allow formation of averages of a plurality of such measurements which are stored in the memory. Typically, 1, 7, 14 or 30 day averages can be formed. Data with special marks such as measurement with a control solution, deleted data, irregular ambient temperature or out of range readings can be disregarded for averaging.

While it is understood that storage of all data typically can be made in one and the same physical memory device, separate memories for different categories of values are conceivable.

The glucose meter can be further provided with a communication interface which allows the exchange of data with an external device such as a display or an external data processing unit. Typically, the data processing unit could be a computer located at a caregiver's premises or a server which can be accessed by a caregiver. This external device can form a system for diabetes management together with the glucose monitor.

The glucose meter according to the present invention or the system can be provided with a graphic display which is adapted for display of a two dimensional graph. This allows displaying e.g. the profile of the glucose level in a function of time. Typically, changes in the glucose level can be followed. In this context, it is particularly advantageous to graphically display the times when the meal time input interface has been activated. If the user activates the input interface each time a meal is taken, the graphic display of the times of activation corresponds to the times of meal intake. An easy correlation between the glucose level and the meal intake therefore is possible. While such operation is preferable in context with a device also having an OGTT operation mode, it is understood that such a display feature could also be advantageous in combination with other devices.

The glucose meter further can have processing means which are adapted to determine an OGTT test result area when the meter is operating in the OGTT mode. The surface defined by the curve of glucose level as a function of time is an important information for a caregiver. By automatically calculating this surface, additional valuable information can be achieved. The OGTT test result area is defined as the area below the curve of the glucose level in function of the time, less the area corresponding to the fasting glucose level.

A further aspect of the invention relates to a method for operating a glucose meter. In this method, in a first step, user instructions to carry out a first measurement are optionally displayed on a user interface of the glucose meter. In a next step, measurement values of the glucose level of a first measurement and the associated time and/or date are stored in a memory of the glucose meter.

After a pre-determined time, user instructions to carry out a second measurement are automatically outputted on the user instruction interface. The measurement values of such second measurement are then stored in the memory of the glucose meter. Operation of a glucose monitor in such a way allows the user to easily make an OGTT measurement.

It is further possible to output user instructions to take a specific amount of glucose after having carried out the first measurement.

According to a further preferred step of the method, the current time and/or date can be stored in the memory upon activation of a meal time input interface which is operatively connected with a processing means of the glucose meter.

It is further possible to repeat the steps of outputting user instructions and storing further measurement results at least once.

It is also possible to form averages of a plurality of measurements of glucose levels which are made at different times, in particular also on different days.

According to another aspect of the invention, a 2D graph which shows the values of a plurality of measurements of the glucose level as a function of the measurement time and the measurement date is displayed. Preferably, times when a meal time input interface has been activated are also registered and displayed.

Furthermore, it is possible to determine an OGTT result area based on the first and at least the second measurement result.

The glucose meter and the method of the present invention provide the user and/or the caregiver with a tool for more reliably measuring glucose levels and therefore for a better management of diabetes.

A further aspect of the invention refers to a computer program product which generates a 2D graph as described above when the program is running on a computer.

The invention now will be explained with reference to a specific embodiment and the accompanying drawings, which show:

Figure 1       an embodiment of a glucose meter of the present invention;

Figure 2       a schematic representation of the separate elements of the glucose meter of the present invention;

Figure 3       a flow chart showing registration of the time of meal intake;

Figure 4       a schematic representation of a display showing the time of meal intake;

Figure 5a      a flow chart of the operation of a glucose meter in OGTT mode;

Figure 5b      an alternative flow chart of the operation of an alternative glucose meter in OGTT mode;

Figure 6       the glucose level displayed as a function of time over 24 hours;

Figure 7       a glucose profile as a function of time after a meal intake;

Figure 8       an OGTT test result area defined by the glucose level as a function of time and;

Figure 9       a schematic representation of OGTT profiles made on different dates.

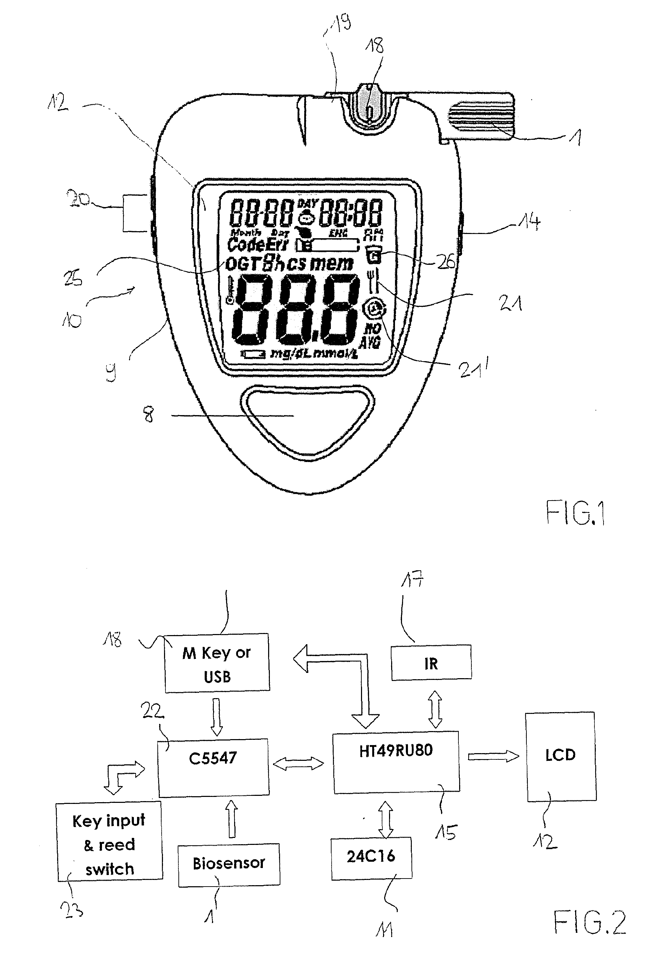

Figure 1 shows a glucose meter 10 with a housing 9. A test strip 1 known to those skilled in the art can be inserted into a test strip port 19 in a manner which is also known to those skilled in the art. The glucose meter 10 is provided with a display 12. The display 12 formed as a LCD display panel is designed to output user instructions in a way which will be disclosed hereinafter. The glucose meter 10 is further provided with an electronic port 18 which can be e.g. a USB connector. This port 18 can be used for data exchange with an external device if no test strip 1 is present in the test strip port 19.

An on/off key 8 allows start/stop operation of the device. In combination with operating keys 20, different functions or menu entries can be selected or made.

The glucose meter 10 is provided with a meal time input interface in the form of a meal key 14. When the meal key 14 is pressed, a fork and knife symbol is displayed in a food display area 21. At the same time, the time and/or date of activation of the meal key 14 is stored in a memory within the glucose meter. This time and/or date is an indication of the time of food intake and is displayed in the time/date field 24.

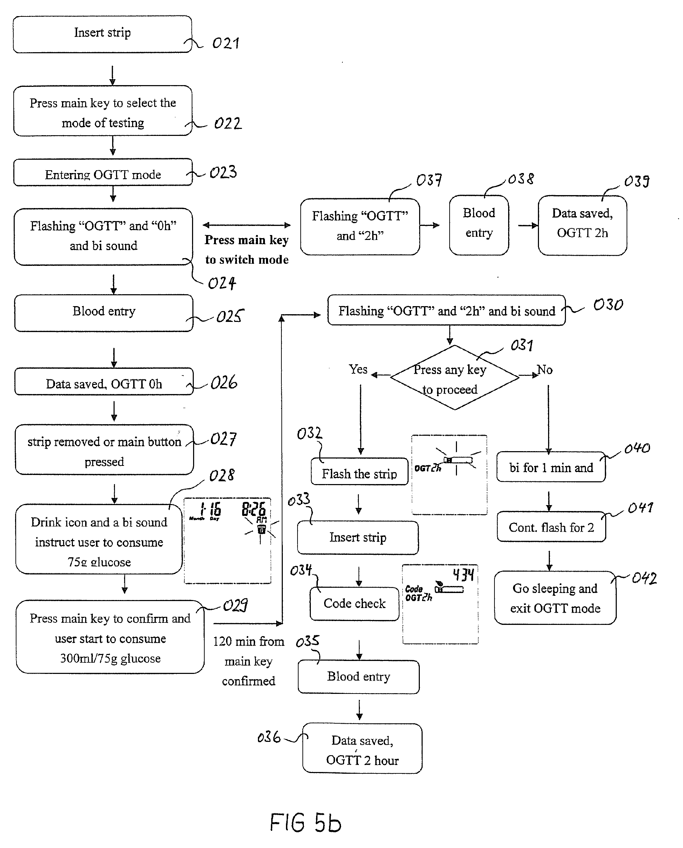

In an OGTT display area 25 information about OGTT measurements (see also Figure 5b) may be displayed. In an OGTT instruction area 26, a user may be instructed to take a standard glucose drink. A plate and cake icon in a meal display area 21' indicates if a specific measurement result has been made before or after a meal intake. In particular, in the meal display area 21' it can be indicated whether a specific glucose measurement has been made after or before meal intake.

In particular, in case of a normal glucose reading lying in the range between 20 mg and 600mg/dl, there can be an indication of the measurement result together with a symbol indicating if the measurement has been made before or after meal intake. The decision criterion may be whether the meal key 14 has been pressed less than two hours before the measurement. If the meal record has been pressed within two hours before the measurement, the glucose result is displayed and marked as "after meal" measurement. If no activation of the meal key 14 has been made within two hours before the measurement, the result is marked as "before meal". The information "after meal" / "before meal" may be stored together with the measurement results and may give the patient further indications about the effect of meal intake to the glucose level.

According to a further preferred embodiment of the invention, it is possible to form averages of glucose measurements. Typically, averages of measurements made on several days can be calculated. It is particularly advantageous to make averages of "before meal" measurements and averages of "after meal" measurements, respectively. This will give the user more reliable information about the effect of meal intake to his or her glucose level.

Typically, measurements of 1, 7, 14 and/or 30 days can be taken into consideration for forming of averages. Since the measurements are stored with their respective time and/or date, averages can also be built based on measurements equally spaced from the time of meal intake. Typically one to three "before meal" and one to three "after meal" measurements are performed during one day.

Figure 2 schematically shows the separate components of the glucose meter 10. A processing means 15, by way of example a HT49RU80 LCD microcontroller unit (provided by Holdtek Semiconductors) can be used. The processing means 15 is in operative connection with an infrared port 17, the LCD display 12, a memory 11, the USB port 18 and a customized multifunctional IC 22 for controlling the key input 23 and for A/D conversion of the signal from the biosensor or test strip 1. The infrared 17 allows the data communication with an external device. The memory 11 allows storing measurement values of the glucose level, the time and/or date associated with such measurement and times of operation of the meal key 14. Instead of an infrared port 17 and an USB port 18, other communication ports, e.g. Bluetooth or FireWire, are also possible.

Switches 23 operated by the user (key switch) or controlled e.g. by magnetic force (reed switch) are used for control of the meter. A user may press the key to activate the meter or to change the operation mode or function of the meter. The reed switch may be used to automatically change the operation mode, e.g. when the device is put into a docking station (not shown) having a magnet and an IR receiver. Automatic data transmission through an IR connection can be performed therewith.

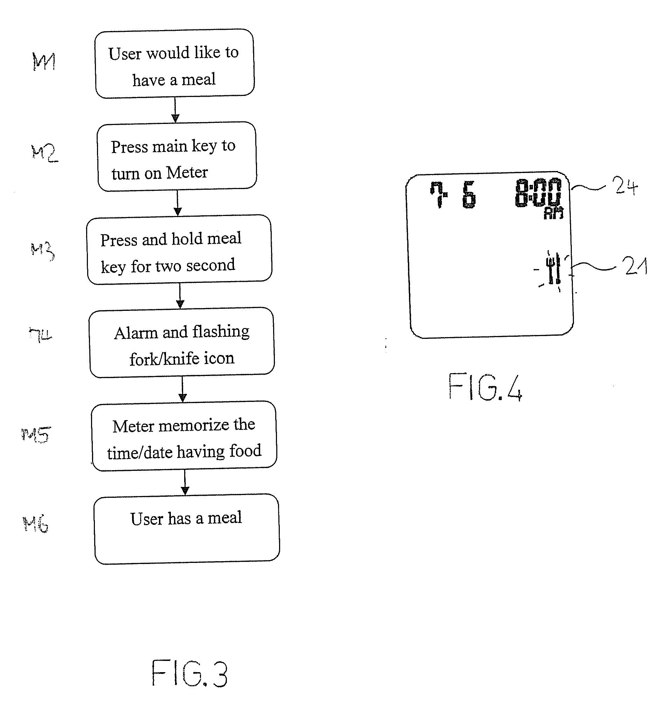

Figure 3 shows the operation of the device in context with activation of the meal key 14. When a user intends to have a meal in a step M1, he presses the main key 8 (see figure 1) in order to turn on the meter (step M2). At the time when the meal is taken, the meal key 14 is pressed and held for about 2 seconds (step M3). As a confirmation, a sound is generated and the fork/knife icon is activated in the display field 21 (step M4). The glucose meter 10 memorises the time and/or date of activation of the meal key 14 and consequently of food intake (step M5). In a final step M6, the user can take a meal. Of course, steps M1 and step M6 are not carried out by the meter and only steps M2 to M5 are in relation with the operation of the glucose meter 10.

The meal time registration shown in figure 4 can be used in context with operation of the glucose monitor 10 in the normal mode, e.g. when no OGTT measurement is carried out. In this normal measurement mode, glucose measurements are made upon insertion of a test strip 1 into the test strip port 19. Each time a test strip 1 is inserted into the test strip port 19, a measurement is made and the respective result of the glucose level and the associated time and/or date is stored in the memory 11 (see figure 2).

By way of example, figure 4 shows a flashing fork/knife symbol in the food display area 21 and the time and/or date of activation of the meal key in the time/date field 24. Selection of the normal and the OGTT mode can be made by means of the operating keys 20 and main key 8 (see figure 1).

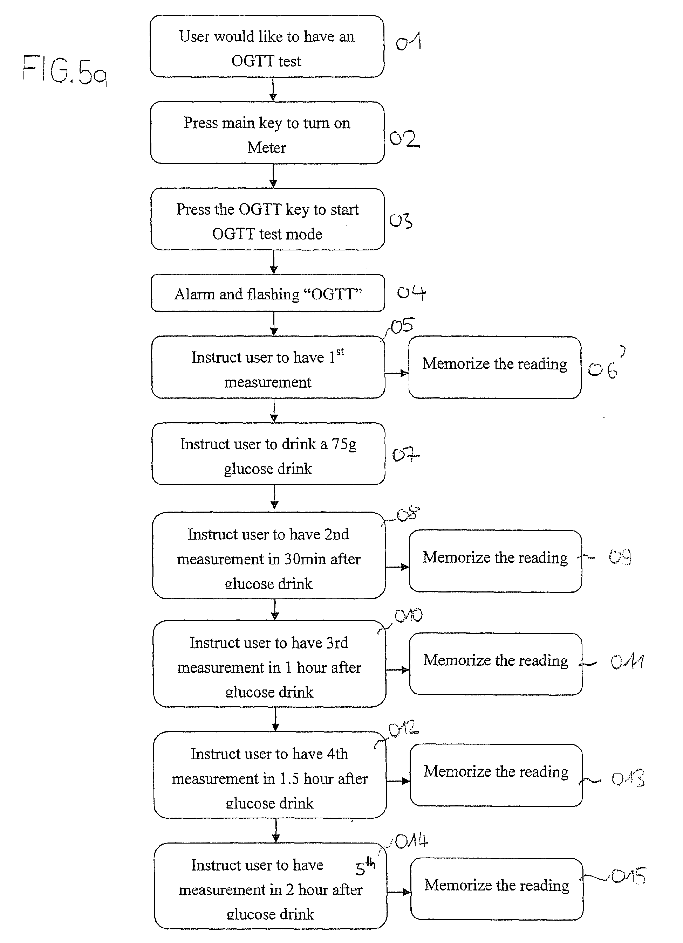

Figure 5a shows operation of a first embodiment of a glucose meter 10 in the OGTT mode. In addition to the meter shown in figure 1, in figure 5a, a separate OGTT key is used to start the OGTT mode. In step O1 a user wishes to have an OGTT made. For this purpose, in step 02, the user presses the main key 8 (see figure 1) in order to turn on the glucose meter. For starting the OGTT mode, the user presses a separate OGTT button not shown in figure 1 (step 03). As a confirmation, in step 04, an OGTT symbol is displayed in an OGTT display area 25 and/or activation of the OGTT mode is confirmed by an alarm.

In step 05, the user is instructed to carry out a first measurement. This instruction is displayed on the display of the glucose meter. Alternatively, audible instructions may be given. By inserting a test strip into the test strip port, a measurement is automatically started. The specific way of measurement is known to those skilled in the art and therefore not explained in more detail in context with the present invention. It may also include entry of specific codes related to the test strip. The values of the glucose measurement are stored in the memory in step 06.

The user is subsequently instructed to drink a 75g glucose drink (step 07). Instruction is made by an appropriate display of the instruction on the display in an OGTT instruction area 26.

In steps 08 to O15, users are instructed to carry out a second, third, fourth and fifth measurement in 30 minute time intervals after the glucose drink intake. Corresponding measurement results of the glucose level are stored in the memory. After the memorisation of the fifth reading in step O15, the OGTT measurement is terminated. Five blood glucose levels and the associated times have been stored in the memory.

It should be noted, that the method shown in the flow chart in figure 5a and the method shown in the flow chart in figure 3 (as a part of a normal measuring mode) are alternative operation modes.

The measurements in the OGTT mode shown in figure 5a are activated by pressing an OGTT button (not shown in figure 1) and by subsequently inserting a measurement strip for each of the measurements.

Figure 5b shows an alternative operation flow chart for an OGTT. This operation is carried out with the device shown in figure 1. Operation of the measurement is started by inserting a measurement strip in a step 021. After insertion of this strip, an operation mode may be selected by using the operation keys 20 and by confirmation of a specific mode by pressing the main key 8. Operation modes are selected in step 022. Operation in an OGTT mode is confirmed by pressing operation key 8 in step 023. In an operation step 024 an OGTT symbol is shown and a time symbol "0h" is displayed in the OGTT display area 25. The time symbol indicates that this in an initial measurement. Display is accompanied by a sound.

In a subsequent step 025, the user is entering blood onto the strip. Measurements are subsequently made and the data of the measurement are saved in step 026. The measurement time "0h" is associated with the saved data. In a further step 027, the strip 1 is removed from the meter 10 or the main button 8 is pressed as an indication that the measurement has been completed. In a next step 028, a drink icon is displayed in the OGTT instruction area 26. The user is thereby reminded to have a drink of 75g glucose. This display may be accompanied by an audible signal.

In step 029, the user confirms intake of 75g glucose/300ml drink by pressing the main key 8.

Two hours after confirmation by the main key 8, the OGTT symbol and a time symbol "2h" are displayed in the OGTT display area 25 in operating step 030.

Operation of the measurement is continued by pressing any key in operation step 031. In step 032 a measurement strip icon is displayed in order to instruct the user to insert a strip. After insertion of this strip in step 033 a code check is made in step 034. Such code checks are known to those skilled in the art in context with blood glucose measuring devices. It is therefore not explained in detail. In step 035 blood is entered onto the strip and measurements are made. Measurement data are saved in step 036, associated with time and/or date information indicating the measurement time "2 hours".

At operation steps 024 by pressing the main key 8, it is possible to change the OGTT mode from "0h" to "2h" 037. By pressing again the main key 8, the OGTT mode is switched back from "2h" to "0h" 024. This configuration allows to perform multiple measurements at "0h", e.g. before intake of the glucose, and at "2h", e.g. 2 hours after glucose intake, to increase the reliability of the measurements. Since always the time and/or date is stored with the measurement results, this further allows to perform multiple measurements at different time intervals, e.g. after 30min, 60min, 90min and after 120min after the glucose intake, which is similar to the operation described with figure 5a. In operating step 037, when entering a blood sample onto the strip a measurement is performed in 038. The measurement data are saved in step 039 together with time and/or date information of the measurement.

If in step 031 no key is pressed, an audible signal is provided during one minute, step 040, and a continuous flashing is indicated on the display for another two minutes in step 041. If no key is pressed within this period of time, the device is switched into a sleep mode and the OGTT mode is automatically ended, step 042.

When operating in the normal mode, a plurality of glucose level measurements are made and stored together with the associated time and/or date. This allows displaying a plurality of glucose level measurements L as a function of time on the display 12 or, alternatively, on an external display or operating unit by means of data exchange through infrared port 17 and/or USB port 18.

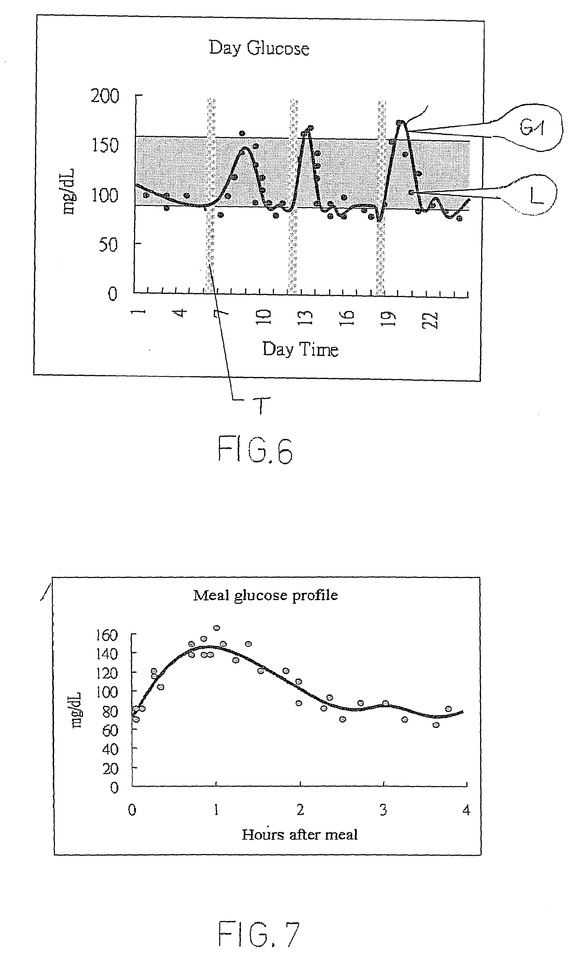

Such a graph displayed on an external device is shown in figure 6. In addition to the individual measurement points L, an approximated graph G1 in the form of an average circadian glucose pattern can be displayed. In the graph of figure 6, each dot L represents a glucose reading.

Furthermore, times of meal intake T may be displayed as a grey column in the graphic representation shown in figure 6. The glucose pattern G1 and the times of meal intake T (corresponding to activation of meal key button 14) allow the verification of the glucose level in context with meal intake.

Figure 7 shows an enlarged view of the glucose profile during the first 4 hours after meal intake.

When operating in the OGTT mode as explained with reference to figure 5a, measurements of the glucose level are made at/before intake of the standard glucose drink, i.e. at the times of fasting and subsequently at 30 minute intervals, at the time of fasting and 2 hours thereafter, or at individual time intervals according to Figure 5b.

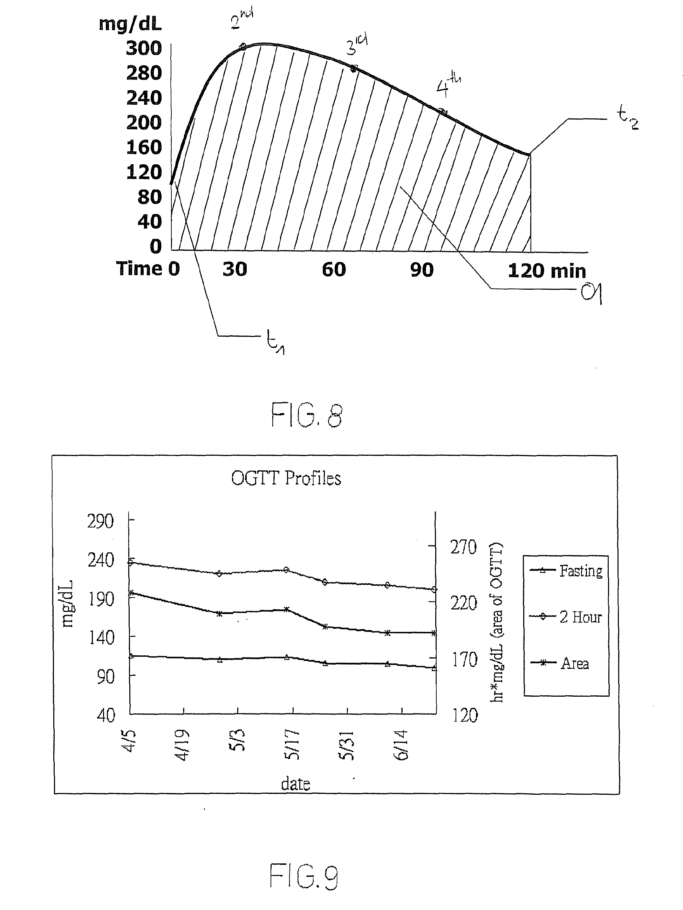

Figure 8 schematically shows a curve of glucose levels measured during such an OGTT mode. The first measurement is made at $t_1$ i.e. at fasting. A second measurement (indicated by a dot) is made after 30 minutes, a third measurement is made after 60 minutes, a fourth measurement is made after 90 minutes and a final measurement is made at time $t_2$ after 120 minutes. The area below the approximated curve running through the measurement values of the first to fifth measurement is defined as the area of OGTT result. The fasting glucose reading taken at $t_1$ can be used as the baseline for an optional determination of the area.

The area can be determined by making an assumption that the curve of the glucose level dependent on the time is a binomial function,

$$y = Ax^2 + Bx + C,$$

whereas
y is the glucose level and x represents the time.

The OGTT result area corresponds to the integral of this function,

$$OGTT1 = \int_{t1}^{t2} y(x)dx = \frac{1}{3}ax^3 + bx^2 + cx,$$

whereas
$t_1$ represents the time of the first and $t_2$ the last i.e. fifth measurement.

The five specific measurement results acquired during the OGTT can be used to fit the curve and to determine the respective values of a, b, and c.

During the OGTT mode, fasting glucose level, glucose level after 2 hours and the OGTT result area OGTT1 are determined. These values can be stored in the memory 11.

Figure 9 shows a graphic display of these three values determined at different dates. The tendency of these values is helpful information for the user and particularly for the caregiver. The display of the area is, however, optional. The OGTT profile allows to assess the risk of a patient suffering from diabetes or also to manage success or efficiency of a therapy or a diet.

Of course, the glucose meter is provided with other means known to those skilled in the art such as power supply, electronic connections and the like which do not need to be explained in detail and which are known to those skilled in the art.

**Claims**

1. A glucose meter (10) for measuring the glucose level in a blood sample of a user, said glucose meter (10) comprising

   - at least one memory (11) for storing at least two measurement results of a glucose level measurement and at least the time and/or date when a first measurement was made
   - a user instruction interface (12) for providing the user with instructions,

   wherein the glucose meter (10) is adapted to be operated in an OGTT operation mode and
   wherein, when operating in said OGTT operation mode, said glucose meter (10) carries out at least the following steps:

   a) optionally outputting on said user instruction interface (12) user instructions to carry out a first measurement of the glucose level
   b) storing the measurement values of said first measurement of the glucose level and the associated time and/or date in said memory (11)
   c) after a predetermined time, automatically outputting on said user instruction interface (12) user instructions to carry out a second measurement
   d) storing the measurement values of the glucose level of said second measurement in said memory (11).

2. A glucose meter (10) according to claim 1, wherein when operating in said OGTT mode, after step b) the glucose meter (10) is adapted to output user instructions on said user instruction interface (11) to take specified amounts of glucose.

3. A glucose meter (10) according to one of the claims 1 or 2,
   wherein the glucose meter (10) is further adapted to operate in a normal measurement mode,

wherein in said normal measurement made one single measurement or a plurality of single measurements of the glucose level is made, and wherein the results of said measurements and the associated times and/or dates of the measurements are stored in said memory (11).

4. A glucose meter (10) according to claim 3 wherein the glucose meter (10) is provided with switching means (23) for switching between said OGTT mode and said normal measurement mode.

5. A glucose meter (10), in particular according to one of the claims 1 to 4, wherein the glucose meter (10) is provided with a meal time input interface (14) operatively connected with a processing means (15) of said glucose meter (10) and wherein the processing means (15) is operating in such a way that upon activation of said meal time input interface (14) the current time and/or date is stored in said memory (11) and wherein preferably the processing means (15) are operating in such a way that information relating to the time of glucose measurement in relation to the time of activation of the meal time input interface is stored in said memory (11).

6. A glucose meter (10) according to one of the claims 1 to 5, wherein the glucose meter (10) is adapted to repeat steps c) and d) at least once when operating in the OGTT mode.

7. A glucose meter (10) according to one of the claims 1 to 6, wherein the memory (11) is adapted to store the values of glucose level measurements made on a plurality of days and wherein the glucose meter (10) preferably comprises processing means (15) for forming averages of a plurality of measurement values stored in said memory (11).

8. A glucose meter (10) according to one of the claims 1 to 7, wherein the glucose meter (10) is provided with a communication interface (17, 18) for exchange of data with a display and/or an external data processing unit.

9. A glucose meter (10) according to one of the claims 1 to 8, wherein the glucose meter (10) is provided with a graphic display (12) or wherein an external display unit with a graphic display is associated with said glucose meter (10), said graphic display (12) allowing two dimensional display of a graph (G1).

10. A glucose meter (10) according to one of the claims 1 to 9, wherein the glucose meter has processing means (15) adapted to determine an OGTT result area (OGTT1) formed below the curve of glucose level as a function of time when operating in said OGTT mode.

11. A glucose monitoring system, in particular with a glucose meter (10) according to one of the claims 1 to 10, wherein the glucose meter (10) is provided with a memory (11) for storing a plurality of measurement results of a glucose level measurement and the associated time and/or date when the measurements were made, the glucose meter (10) further comprising a meal time input interface (14) operatively connected to a processing means (15) of the glucose meter (10), wherein the processing means (15) is operating in such a way that upon activation of said meal key (14) the current time and/or date is stored in said memory (11), wherein the glucose meter (10) has a graphic display (12) or an external display unit connected to or connectable to said glucose meter (10) adapted to display a graph (G1) showing a value of a plurality of measurements as a function of the measurement time and/or date, and wherein preferably the processing means (15) or said external device are adapted to graphically display on said graph (G1) the times when said meal time input interface (14) was activated.

12. A method for operating a glucose meter (10), the method comprising the steps of

   a) optionally outputting on a user interface (12) of said glucose meter (10) user instructions to carry out a first measurement
   b) storing the measurement values of the glucose level and the associated time and/or date of said measurement in a memory (11) of said glucose meter (10)
   c) after a pre-determined time, automatically outputting on said user instruction interface (12) user instructions to carry out a second measurement
   d) storing the measurement values of said second measurement of the glucose level in said memory (11).

13. A method according to claim 12, comprising the further step of automatically outputting user instructions to take a specified amount of glucose after step b.

14. A glucose meter (10) according to one of the claims 12 or 13, comprising the further step of storing the current time and/or date in a memory (11) upon activation of a meal time input interface (14) operatively connected to a processing means (15) of said glucose meter (10).

15. A method according to one of the claims 12 to 14, wherein step c) and d) are repeated at least once.

16. A method according to one of the claims 12 to 15, comprising the further step of forming an average of a plurality of measurement values stored in said memory (11).

17. A method according to one of the claims 12 to 16, comprising the further step of displaying a graph (G1) showing the values of a plurality of measurements of the glucose level as a function of the measurement time and/or date and
preferably displaying the times and/or dates when said meal input interface (14) was activated.

18. A method according to one of the claims 12 to 16, comprising the further step of determining an OGTT result area (OGTT1) based on at least the first and second measurement result acquired during the OGTT mode.

19. A method for managing diabetes care with a glucose meter (10), comprising the steps of

   a) storing the current time and/or date when a meal key (14) of the glucose meter (10) is activated
   b) measuring glucose levels of a blood sample and storing the result of said measurements and the associated time and/or date in a memory (11) of the glucose meter (10)
   c) displaying in a two dimensional graphic form (G1), the glucose levels measured as a function of the time of measurement
   d) displaying, in the same graphical representation (G1), the times when the meal time input instruction interface (14) was activated.

20. A computer program product carrying out the display steps of c) and d) of claim 18 when the computer program product is running on an external device connected to or connectable to a glucose meter according to claim 11.

FIG.1

| M Key or USB | | IR | |
|---|---|---|---|
| C5547 | HT49RU80 | | LCD |
| Key input & reed switch | | 24C16 | |
| Biosensor | | | |

FIG.2

M1 → User would like to have a meal

M2 → Press main key to turn on Meter

M3 → Press and hold meal key for two second

M4 → Alarm and flashing fork/knife icon

M5 → Meter memorize the time/date having food

M6 → User has a meal

FIG.3

7 6  8:00 AM ~24

~21

FIG.4

FIG.5a

```
┌─────────────────────────┐
│  User would like to have │  o1
│       an OGTT test       │
└─────────────────────────┘
            │
            ▼
┌─────────────────────────┐
│  Press main key to turn  │  o2
│         on Meter         │
└─────────────────────────┘
            │
            ▼
┌─────────────────────────┐
│  Press the OGTT key to   │  o3
│   start OGTT test mode   │
└─────────────────────────┘
            │
            ▼
┌─────────────────────────┐
│ Alarm and flashing "OGTT"│  o4
└─────────────────────────┘
            │
            ▼                       o5
┌─────────────────────────┐      ┌──────────────────────┐
│   Instruct user to have  │─────▶│  Memorize the reading │  o6
│      1st measurement     │      └──────────────────────┘
└─────────────────────────┘
            │
            ▼
┌─────────────────────────┐
│  Instruct user to drink  │  o7
│     a 75g glucose drink  │
└─────────────────────────┘
            │
            ▼                       o8
┌─────────────────────────┐      ┌──────────────────────┐
│  Instruct user to have   │─────▶│  Memorize the reading │  o9
│  2nd measurement in 30min│      └──────────────────────┘
│   after glucose drink    │
└─────────────────────────┘
            │
            ▼                       o10
┌─────────────────────────┐      ┌──────────────────────┐
│  Instruct user to have   │─────▶│  Memorize the reading │  o11
│  3rd measurement in 1    │      └──────────────────────┘
│  hour after glucose drink│
└─────────────────────────┘
            │
            ▼                       o12
┌─────────────────────────┐      ┌──────────────────────┐
│  Instruct user to have   │─────▶│  Memorize the reading │  o13
│ 4th measurement in 1.5   │      └──────────────────────┘
│ hour after glucose drink │
└─────────────────────────┘
            │
            ▼                       o14
┌─────────────────────────┐      ┌──────────────────────┐
│  Instruct user to have   │─────▶│  Memorize the reading │  o15
│ 5th measurement in 2     │      └──────────────────────┘
│ hour after glucose drink │
└─────────────────────────┘
```

Insert strip — 021

Press main key to select the mode of testing — 022

Entering OGTT mode — 023

Flashing "OGTT" and "0h" and bi sound — 024

**Press main key to switch mode**

Flashing "OGTT" and "2h" — 037

Blood entry — 038

Data saved, OGTT 2h — 039

Blood entry — 025

Flashing "OGTT" and "2h" and bi sound — 030

Data saved, OGTT 0h — 026

Press any key to proceed — 031

Yes / No

strip removed or main button pressed — 027

Drink icon and a bi sound instruct user to consume 75g glucose — 028

Press main key to confirm and user start to consume 300ml/75g glucose — 029

Flash the strip — 032

OGT 2h

Insert strip — 033

Code check — 034

Code OGT 2h

434

120 min from main key confirmed

Blood entry — 035

036 — Data saved, OGTT 2 hour

bi for 1 min and — 040

Cont. flash for 2 — 041

Go sleeping and exit OGTT mode — 042

FIG 5b

FIG.6

FIG. 7

14

FIG. 8

FIG. 9

Europäisches
Patentamt
European
Patent Office
Office européen
des brevets

# EUROPEAN SEARCH REPORT

Application Number

EP 08 10 4228

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/132376 A (KONINKL PHILIPS ELECTRONICS NV [NL]; BODLAENDER MAARTEN P [NL]; STUT W) 22 November 2007 (2007-11-22) * page 7, lines 4-9 * * page 9, line 32 - page 10, line 12 * * page 11, lines 11,12 * * page 15, lines 14,15 * * page 17, lines 5,6,22-32 * * page 27, line 19 - page 29, line 34; figure 6 * | 1-4,6-8, 12-17 | INV. A61B5/00 G06F19/00 G01N33/487 |
| X | US 2007/276209 A1 (EMOTO FUMIAKI [JP] ET AL) 29 November 2007 (2007-11-29) * abstract * * paragraphs [0011] - [0041] * * paragraphs [0064], [0068], [0072], [0074], [0080] - [0088], [0114] * | 1,6-10, 15-18 | |
| X | EP 1 918 837 A (HOFFMANN LA ROCHE [CH]; ROCHE DIAGNOSTICS GMBH [DE]) 7 May 2008 (2008-05-07) | 5,11,19, 20 | |
| A | * abstract; figures 8-12 *  * paragraphs [0001], [0008], [0009], [0012], [0013], [0021], [0024], [0025], [0028], [0035] - [0037], [0054], [0057], [0069], [0070], [0073] * | 9,10,17, 18 | TECHNICAL FIELDS SEARCHED (IPC) A61B G06F G01N |
| X | WO 03/057027 A (MEDTRONIC MINIMED INC [US]) 17 July 2003 (2003-07-17) * abstract * * paragraphs [0022], [0039], [0040] * | 5 | |
| A | * paragraphs [0090] - [0098]; figure 6D * | 9-11,17, 18 | |

-/--

The present search report has been drawn up for all claims

| Place of search | Date of completion of the search | Examiner |
|---|---|---|
| Munich | 14 January 2009 | Medeiros Gaspar, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                           

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 08 10 4228

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2007/019289 A (BAYER HEALTHCARE LLC [US]; POWER BARRY D [US]; CULVER JEFFREY A [US]) 15 February 2007 (2007-02-15) * abstract * * paragraphs [0013], [0014], [0018], [0021]; figure 3d * ----- | 5,7,16 | |
| A | WO 2006/072035 A (LIFESCAN SCOTLAND LTD [GB]; YOUNG STANLEY ALAN [GB]; TAYLOR DAVID WILL) 6 July 2006 (2006-07-06) * abstract * * paragraphs [0001], [0044] - [0046], [0052]; figure 14 * ----- | 5 | |

TECHNICAL FIELDS
SEARCHED    (IPC)

The present search report has been drawn up for all claims

| Place of search | Date of completion of the search | Examiner |
|---|---|---|
| Munich | 14 January 2009 | Medeiros Gaspar, Ana |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&  : member of the same patent family, corresponding
    document

EPO FORM 1503 03.82 (P04C01)

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 08 10 4228

The Search Division considers that the present European patentapplication does not comply with the requirements of unity of invention and relates to severalinventions or groups of inventions, namely:

1. claims: 1-4,6-10,12-18

      glucose meters adapted to be operated in an OGTT operation mode, and methods thereof
                                    ---

2. claims: 5,11,19,20

      glucose meters comprising a meal time input interface allowing for storage of information concerning the times of intake of food, and corresponding methods and computer program product
                                    ---

**ANNEX TO THE EUROPEAN SEARCH REPORT
ON EUROPEAN PATENT APPLICATION NO.**

EP 08 10 4228

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2009

| Patent document cited in search report | | Publication date | Patent family member(s) | | Publication date |
|---|---|---|---|---|---|
| WO 2007132376 | A | 22-11-2007 | NONE | | |
| US 2007276209 | A1 | 29-11-2007 | WO | 2005106446 A1 | 10-11-2005 |
| EP 1918837 | A | 07-05-2008 | WO | 2008052374 A1 | 08-05-2008 |
| WO 03057027 | A | 17-07-2003 | AU | 2002360634 A1 | 24-07-2003 |
| | | | EP | 1460931 A2 | 29-09-2004 |
| | | | US | 2003125612 A1 | 03-07-2003 |
| | | | US | 2008255438 A1 | 16-10-2008 |
| | | | US | 2005027182 A1 | 03-02-2005 |
| | | | US | 2005113653 A1 | 26-05-2005 |
| | | | US | 2005096511 A1 | 05-05-2005 |
| | | | US | 2005096512 A1 | 05-05-2005 |
| WO 2007019289 | A | 15-02-2007 | AR | 054899 A1 | 25-07-2007 |
| | | | AU | 2006278537 A1 | 15-02-2007 |
| | | | CA | 2617965 A1 | 15-02-2007 |
| | | | CN | 101238368 A | 06-08-2008 |
| | | | EP | 1913383 A1 | 23-04-2008 |
| | | | KR | 20080034979 A | 22-04-2008 |
| | | | NO | 20081165 B | 05-05-2008 |
| | | | UY | 29720 A1 | 30-03-2007 |
| WO 2006072035 | A | 06-07-2006 | CA | 2593376 A1 | 06-07-2006 |
| | | | CN | 101137320 A | 05-03-2008 |
| | | | EP | 1835849 A1 | 26-09-2007 |
| | | | JP | 2008526320 T | 24-07-2008 |

EPO FORM P0459

For more details about this annex : see Official Journal of the European Patent Office, No. 12/82

REFERENCES CITED IN THE DESCRIPTION

**Patent documents cited in the description**

- WO 2007019384 A
- EP 1733677 A
- US 20030211617 A
- US 7241256 B
- US 7228163 B
- WO 9109139 A